# EUROPEAN PATENT SPECIFICATION

(11) **EP 1 816 985 B1**
(45) Date of publication and mention of the grant of the patent: **19.12.2012**
(21) Application number: 05849636.5
(22) Date of filing: 17.11.2005
(51) Int. Cl.: A61F 2/16

(54) **BIOCOMPATIBLE POLYMERIC COMPOSITIONS FOR USE IN MAKING POSTERIOR CHAMBER INTRAOCULAR LENSES**
BIOLOGISCH VERTRÄGLICHE POLYMERZUSAMMENSETZUNGEN ZUR VERWENDUNG BEI DER HERSTELLUNG VON INTRAOKULARER LINSEN DER HINTEREN AUGENKAMMER
COMPOSITIONS POLYMERES BIOCOMPATIBLES A UTILISER DANS LA FABRICATION DE CRISTALLINS ARTIFICIELS DE CHAMBRE POSTERIEURE

(30) Priority: 30.11.2004 US 632148 P
(43) Date of publication of application: 15.08.2007
(73) Proprietor: Abbott Medical Optics Inc., Santa Ana, CA 92705-4933 (US)
(72) Inventor: MAKKER, Harish, C., Mission Viejo, CA 92691 (US); GHAZIZADEH, Massoud, Laguna Niguel, CA 92677 (US); LOWERY, Michael, D., Vista, CA 92081 (US)
(74) Representative: HOFFMANN EITLE
(86) International application number: PCT/US2005/041776
(87) International publication number: WO 2006/060179

(56) References cited:
- EP-A- 0 898 972
- WO-A-2005/066662
- US-A- 5 221 073
- US-A1- 2003 193 100
- US-B1- 6 277 940

## Description

### FIELD OF THE INVENTION

The present invention generally relates to biocompatible polymeric compositions. Specifically, the biocompatible polymeric compositions of the present invention are useful for fabricating intraocular lenses (IOL). More specifically the biocompatible polymeric compositions are intended for making posterior chamber intraocular lenses.

### BACKGROUND OF THE INVENTION

Intraocular lenses (IOLs) were first used as a replacement for damaged natural crystalline lenses in 1949. These early IOLs were implanted into the posterior chamber after the natural crystalline lens was surgically removed. The first physician to use posterior chamber IOLs as replacements for the natural crystalline lens was English RAF ophthalmologist Dr. Howard Ridley. Dr. Ridley first observed acrylate polymer biocompatibility in the eyes of pilots who had sustained ocular injuries from polymethylmethacrylate (PMMA) shards when their aircraft canopies were shattered. However, it took nearly thirty years for ophthalmologists to embrace IOL implantation as a routine method for restoring vision in patients suffering from diseased or damaged natural crystalline lenses.

Early IOLs were made from PMMA because of its proven biocompatibility. Polymethylmethacrylate is a ridged polymer and requires a 5 mm to 7 mm incision. Incision size is directly related to patient trauma, discomfort and healing times. Moreover, incisions sizes in the 5 mm to 7 mm range generally require sutures further increasing procedural complexity and patent discomfort. Lens size dictates incision size and lens size is in turn determined by the size of the capsular sac and natural crystalline lens. Thus lenses made from a rigid polymer such as PMMA require an incision size at least as large as the minimum IOL dimension which is generally 5.5 mm on average.

In an effort to decrease incision size and corresponding patient discomfort, recovery time and procedural complexity, a number of IOL designs suitable for insertion through small incisions have been developed; most notably foldable IOLs. Foldable IOLs are made from non-rigid, or pliable polymers including hydrophobic acrylics, hydrophilic hydrogels, silicone elastomers and porcine collagen. Intraocular lenses made from these materials can be folded or rolled into implantable configurations having minimum dimensions suited for 3 mm incisions, or less.

Intraocular lenses are used to restore vision to patients having damaged natural crystalline lenses or replace the natural lens when warranted by medical conditions. This generally involved implanting a polymeric IOL into the capsular sac in the eye's posterior chamber after the damaged natural crystalline lens was surgically removed. Recently, refractive correction using IOLs in the phakic eye, that is an eye which retains the natural lens, has grown in popularity as an option to refractive laser surgery. However, there are difficulties associated with implanting an IOL in the phakic eye that are not encountered when implanting a lens in the aphakic eye, that is an eye in which the natural lens has been removed. The phakic eye is a substantially more reactive environment than the aphakic eye. Inflammatory reactions tend to be greater in the phakic eye resulting in a concomitant increase in damage to the eye caused by implanting intraocular lenses. Moreover, the presence of the natural lens in the phakic eye significantly reduces the space available for posterior chamber implantation. Thus, an IOL implanted into the posterior chamber of the phakic eye will directly contact the posterior surface of the natural crystalline lens.

EP 0 898 972 A2 describes a soft intraocular lens material which consists essentially of a polymer obtained by polymerizing polymerizable components containing a hydrophilic monomer, and which has a water absorptivity of from 1.5 to 4.5 wt.%. This document does not disclose a combination of ethyl acrylate and ethyl methacrylate as polymerizable components.

US 2003/0193100 A1 describes an intraocular lens comprising a copolymer obtained by copolymerization of predetermined amounts of 2-[2-(perfluorooctyl]ethoxy]-1-methylethyl (meth)acrylate, 2-phenylethyl (meth)acrylate, alkyl (meth)acrylate and a crosslinking monomer.

US 5,331,073 describes a high refractive index polymeric composition and foldable intraocular lenses made from such composition. The composition comprises a copolymer including a major amount of a first constituent derived from a first monomeric component, a minor amount of a second constituent derived from a second monomeric component other than the first monomeric component, and a third constituent derived from a crosslinking monomeric component.

US 6,277,940 B1 relates to a material for a soft intraocular lens, said material comprising 5 - 25 % by weight of acrylate containing an aromatic ring, 50 - 90 % by weight of 2-hydroxyethyl (meth)acrylate, and 5 - 45 % by weight of a (meth)acrylate monomer.

WO 2005/066662 A1 constitutes prior art pursuant to Article 54 (3) EPC and describes a polymer material useful for manufacturing an intraocular lens and comprising specific acrylic-silicone hybrids.

Therefore, there is a need for biocompatible polymeric compositions that can be used to make posterior chamber IOLs that are thin and pliable enough to fit easily through small incisions, have sufficient mechanical strength to resist impact-related damage and can be made in a wide range of diopters sufficient to provide refractive correction for myopia, hyperopia, presbyopia, astigmatisms and for implantation after removal of the natural crystalline lens as warranted by medical conditions such as cataracts.

### SUMMARY OF THE INVENTION

The present invention is directed to intraocular lenses, specifically intraocular lenses (IOL) suitable for placement in the posterior chamber of the phakic or aphakic eye. The posterior chamber intraocular lenses (PC-IOL) of the present invention are intended for refractive correction and are suitable for correcting myopia, hyperopia, presbyopia, astigmatisms and for implantation after removal of the natural crystalline lens as warranted by medical conditions such as cataracts.

In the present invention, a biocompatible polymer is provided comprising 52 mass percent to 56 mass percent of a first alkyl acrylate, 18 mass percent to 22 mass percent of a second alkyl acrylate, 24 mass percent to 28 mass percent of a third alkyl acrylate, mass percent to 5 mass percent of a diacrylate ester crosslinking agent wherein the biocompatible polymer is used to form a posterior chamber intraocular lens (PC-IOL). The first alkyl acrylate, second alkyl acrylate and third alkyl acrylate are phenoxyethyl acrylate, ethyl acrylate and ethyl methacrylate, respectively. Moreover, the diacrylate ester crosslinking agent used to make the PC-IOLs of the present invention are selected from the group consisting of ethylene glycol dimethacrylate, propylene glycol dimethacrylate, ethylene glycol diacrylate and combinations thereof.

In another embodiment of the present invention, a PC-IOL is provided consisting essentially of 54 mass percent of phenoxyethyl acrylate, 20 mass percent of ethyl acrylate, 26 mass percent of ethyl methacrylate, and 4 mass percent of ethyleneglycol dimethacrylate wherein residual solvents and UV absorbing compounds make up the remaining mass percentage such that the total mass percent is 100.

In yet another embodiment of the present invention, said biocompatible polymer has a tensile strength 10.75 MPa (1559 psi); an elongation at break of 97% and is used to form a PC-IOL. Moreover the PC-IOLs made in accordance with the teachings of the present invention have a refractive index (n_{D}) at 20°C-25°C of between approximately 1.45 and 1.55. In a preferred embodiment the PC-IOL has a refractive index (n_{D}) at 20°C-25°C of approximately 1.52.

### DEFINITION OF TERMS

To aid in the understanding the following detailed description of the present invention, the terms and phases used herein shall have the following, non-limiting, definitions.

Aphakic: As used herein "aphakic" shall mean the condition where the natural crystalline lens has been removed form the eye, that is, an eye lacking its natural crystalline lens.

Mass percent: As used herein "mass percent" is defined as the mass of the solute in grams multiplied by 100 divided by the mass of the solution in grams i.e. mass % = mass of solute (in grams) (100) / mass of solution (in grams).

Mechanical strength: "Mechanical strength" is a subjective terms and as used herein refers to the sum of a polymer's physical properties that define a polymer's resiliency. Specifically, as used herein "mechanical strength" refers to the polymer's ability to resist tearing. Thus a polymer having suitable mechanical strength as defined herein will result in an IOL that deforms sufficiently to absorb impact stress yet does not tear. Moreover, the IOL will then quickly return to its pre-stressed shape after the source of the impact stress has been removed. As used herein an IOL made from a polymer having inadequate mechanical strength will result in a lens that is slow to rebound and return to its pre-stressed shape and is more prone to tear when stressed. In contrast, an IOL having to made from a polymer having too great of a mechanical strength will make the lens too rigid, or "stiff" and less responsive to stress and thus more prone to maintain its pre-stressed shape under strain and cause injury to the eye's delicate structures. Moreover, excessively rigid lens cannot be folded, rolled or otherwise sufficiently deformed to be inserted through small incisions.

Pliable: As used herein "pliable" means "flexible" and refers to a polymeric IOL that can be folded, rolled or otherwise deformed sufficiently to be inserted through a small incision.

Phakic: As used herein "phakic" refers to an eye having the natural crystalline lens in place.

Residual solvents: As used herein "residual solvent(s)" refers to trace solvents that may be present in the polymer matrix after the PC-IOL formed from the solvents have been processed and are in final form suitable for deployment into the eye.

Resiliency: As used herein "resiliency" refers to a polymeric IOL having sufficient mechanical strength to return to its pre-stressed configuration following impact and the resulting deformation associated with the stress on impact, also referred to herein after as "rebound resiliency."

Softness: As used herein "softness" refers to a polymeric IOL that is resilient and pliable as opposed to a polymethylmethacrylate (PMMA) IOL that is rigid and hard.

Small incision: As used herein the term "small incision" refers to a surgical incision of less than approximately 5 mm made in the eye's cornea that permits the insertion of an IOL into the eye. Preferably the incision is less that 3 mm and even more preferably the incision is less than 2 mm.

### DETAILED DESCRIPTION OF THE INVENTION

The present invention is directed to intraocular lenses, specifically intraocular lenses (IOL) suitable for placement in the posterior chamber of the phakic or aphakic eye. Traditional intraocular lenses are available in a wide range of biocompatible materials ranging from hard plastic compositions such as polymethylmethacrylate (PMMA) to soft highly flexible materials including silicones, certain acrylics and hydrogels. Recently the more pliable, or softer lenses have gained in popularity due to their ability to be compressed, folded, rolled and otherwise deformed. These more pliable IOLs can be inserted through much narrower incisions than hard PMMA lenses and thus reduce the healing time and discomfort associated with IOL implantation.

The majority of IOL procedures involve inserting an IOL into the posterior chamber (PC) or anterior chamber (AC) of an aphakic eye as a replacement for a damaged or diseased natural crystalline lens that has been surgically removed from the eye. While these lenses also possess refractive corrections, the primary purpose is to restore sight lost to the damaged or diseased natural lens. However, surgically implanted IOLs as a permanent form of refractive correction have recently gained popularity.

The IOLs of the present invention must be sufficiently pliable for small incision implantation and also resilient enough to recover quickly when deformed in the eye as the result of incidental contact. Moreover, in order to minimize patient discomfort and decrease recovery time, it is desirable to insert the IOL through a small incision, preferably a 3 mm incision or less. This requires that the lens be pliable so that it easily deforms to reduce the pre-insertion size and yet resilient enough to gently unfold once implanted. However, because the IOL of the present invention must also be thin enough to provide a suitable fit within the eye's posterior chamber, the material used to fabricate the IOL must have sufficient mechanical strength to prevent the pliable IOL from tearing during implantation or use.

Therefore, the present invention provides polymeric compositions that balance the competing physical properties described above; namely, the polymer compositions of the present inventive are biocompatible, are pliable enough to be folded rolled or otherwise deformed sufficiently to be inserted through small incisions, possess sufficient mechanical strength that they can be shaped thin and yet have sufficient mechanical strength to provide rebound resiliency upon impact without tearing.

The biocompatible polymers of the present invention are useful for the fabrication of PC-IOLs having the properties defined above. The present inventors have developed the disclosed biocompatible polymers specifically to achieve a pliable, resilient and durable PC-IOL that can be shaped to achieve refractive correction for a wide range of vision anomalies including myopia, hyperopia, presbyopia, astigmatisms and for implantation after cataract surgery. It is desirable to have an IOL that can be folded, rolled or otherwise deformed such that it can be inserted through a small incision in order to minimize patient trauma and post surgical recovery time. Thus, a thin, pliable polymeric IOL is desirable.

In one embodiment of the present invention a biocompatible polymer is provided comprising 52 mass percent to 56 mass percent of a first alkyl acrylate, 18 mass percent to 22 mass percent of a second alkyl acrylate, 24 mass percent to 28 mass percent of a third alkyl acrylate, 3 mass percent to 5 mass percent of a diacrylate ester crosslinking agent wherein the biocompatible polymer is used to form a PC-IOL. The first alkyl acrylate, second alkyl acrylate and third alkyl acrylate are phenoxyethyl acrylate, ethyl acrylate and ethyl methacrytate, respectively. Moreover, the diacrylate ester crosslinking agent used to make the PC-IOLs of the present invention are selected from the group including, but not limited to, ethylene glycol dimethacrylate, propylene glycol dimethacrylate, ethylene glycol diacrylate and combinations thereof.

In another embodiment of the present invention a PC-IOL is provided consisting essentially of 54 mass percent of phenoxyethyl acrylate, 20 mass percent of ethyl acrylate, 26 mass percent of ethyl methacrylate, and 4 mass percent of ethyleneglycol dimethacrylate wherein residual solvents and UV absorbing compounds make up the remaining mass percentage such that the total mass percent is 100.

In yet another embodiment of the present invention, the biocompatible polymer has a tensile strength of 10.75 MPa (1559 psi); an elongation at break of 97% and is used to form a PC-IOL. Moreover the PC-IOLs made in accordance with the teachings of the present invention have a refractive index (n_{D}) at 20°C-25°C of between approximately 1.45 and 1.55. In a preferred embodiment the PC-IOL has a refractive index (n_{D}) at 20°C-25°C of approximately 1.52.

It is understood by those having ordinary skill in the art that other methods of synthetic polymer chemistry may be used to achieve the biocompatible polymeric compositions of the present invention and as such the following process is non-limiting. Moreover, persons having ordinary skill in the art will recognize that the materials used in the following process are readily available from many different commercial sources. Therefore, the source of the materials used herein is not limiting.

In one embodiment, the polymeric compositions of the present invention begin with preparing a reaction mixture having approximately 52 mass percent to 56 mass percent phenoxyethyl acrylate, approximately 24 mass percent to 28 mass percent ethyl methacrylate and ethyl acrylate in a weight percent concentration of approximately 18 mass percent to 22 mass percent. In the reaction mixture, the n-butyl acrylate or ethyl acrylate provides flexibility in the presence of methacrylate esters principally because of the low glass transition temperature thereof. However, the ethyl acrylate renders the mixture tacky or sticky. In addition to the foregoing, the reaction mixture may also include at least one an ultraviolet (UV) light absorber such as, but not limited to, the UV chromophores benzophenones and benzotriazoles-based compounds (for example UV Absorbing Material, UVAM) and/or a blue-violet light absorbing dye such as but not limited to methine and azo class yellow dyes disclosed in co-pending United States Provisional Patent Application Numbers 60/629,556 and 60/629,557, both filed November 22, 2004, regarding polymerizable ultraviolet light absorbing methine and anthraquinone compounds, specifically Examples 1-136 on pages 28-60 of Application No. 60/629,556 and Examples 1-87 on pages 27-47 of Application No. 60/629,557. In some embodiments a free radical initiator such as, but not limited to, an aliphatic peroxide may also be included. The UV-absorber, blue-violet light absorbing dye and initiator are present at from approximately 0.05% to 5.0% by weight concentrations. The reaction mixture also includes at least one initiator and at least one cross-linking agent such as a diacrylate ester. The type and amount of cross-linking agent is carefully selected to obtain the requisite degree of mechanical strength and pliability.

In one method for making the biocompatible polymers for the present invention a reaction mixture is prepared in a suitable reaction vessel such as a one liter three-neck round-bottom flask by carefully mixing approximately 52 to 56 weight percent phenoxyethyl acrylate (PEA), approximately 24 to 28 weight percent ethyl methacrylate (EMA), approximately 18 to 22 weight percent ethylacrylate (EA), approximately 3 to 5 weight percent ethyleneglycol dimethacrylate (EGDMA), approximately 0.10 to 0.50 weight percent of a suitable thermal initiator, such as a peroxide including but not limited to di-tert-butyl peroxide (Trigonox^{®} a registered trademark of Akzo Chemie Nederland B.V. Corporation Amersfoort, Netherlands) or 2,5-dimethyl-2,5-bis (2-ethylhexanoylperoxy) hexane and approximately 0.5 weight percent of UVAM. The thermal imitator is generally added last after the reaction vessel is securely supported and provided with a mixing means such as a magnetic stir plate with stir bar or a low-shear impellor and overhead drive. Next nitrogen gas is gently (6.3 MPa; 1 PSI) bubbled through the reaction mixture for approximately 15 minutes and the reaction mixture is degassed under vacuum (11.7±0.3 kPa approximately 88 ± 2 Torr) for five minutes. Because thermal initiated polymerization is exothermic it is important to maintain control over the reaction mixture. An immersion chiller water bath can be used to prevent the reaction mixture from overheating.

The IOLs of the present invention are formed by transferring the biocompatible polymer reaction mixture into molds having the desired shape before the polymerization and cross-linking reactions are complete. In one embodiment of the present invention, molds are provided to receive the liquid reaction mixture. The molds are first brought to a suitable temperature that permits the polymer lens to cure in a controlled manner. In one embodiment of the present invention a water bath is used to maintain mold temperature at approximately 78°C ± 2°C. One non-limiting method for transferring the reaction mixture to the molds is by increasing the pressure in the reaction vessel relative to atmospheric pressure and providing a route for the pressurized reaction mixture to exit the reaction vessel. In one embodiment of the present invention nitrogen gas is pumped into the reaction vessel and the reaction mixture is forced from the reaction vessel through an appropriate grade of tubing. As the reaction mixture exits the reaction vessel it is passed though a filter into the mold. The filled mold is then maintained at approximately 78°C ± 2°C for 18 to 24 hours. Next the molds are transferred to a dry heat curing oven equilibrated to approximately 90°C. The molds are held at this temperature for an additional 22 to 24 hours. At this point solid, soft acrylic polymer sheets are now ready to be processed further to form IOLs having various diopters as known to those skilled in the art.

The materials used to prepare a preferred embodiment of the present invention are summarized in the following table:

| **Polymer Ingredient** | **Mass Percent¹** |
|---|---|
| Phenoxyethyl Acrylate (PEA) | 54.0 |
| Ethyl Acrylate (EA) | 20.0 |
| Ethyl Methacrylate (EMA) | 26.0 |
| Ethyleneglycol dimethacrylate (EGDMA) | 4.0 |
| Trigonox^{®} 141 (Thermal initiator) | 0.30 |
| Trigonox^{®} C (Thermal initiator) | 0.15 |
| WAM | 0.5 |

| | |
|---|---|
| ¹ Mass percents may not total to exactly 100% due to rounding errors. | |

The biocompatible polymeric materials made in accordance with the teachings of the present invention suitable for use in fabricating IOLs should possess the following physical characteristics:

| | |
|---|---|
| Tensile Strength [MPa (PSI)]² | 10.75 ± 0.43 (1559±62) |
| Elongation at Break (%)² | 97 ± 0 |
| Modulus | 2691 ± 86 |
| Refractive Index | 1.5203 ± 0.0001 |

| | |
|---|---|
| ² Methods and instrumentation for mechanical property (Tensile, % Elongation at Break) determinations as expressed herein include: Instrument = MTS tester. Sample Die = ASTM D412 "C" Temperature = 20-25°C Pull Rate = 20 inches/minute Number of Samples Averaged = 9 per test condition | |

In a preferred embodiment the biocompatible polymer of the present invention possesses the following physical characteristics: Tensile Strength of 10.75 MPa (1559 psi); Percent Elongation at Break of 97%; Modulus of 2691; and Refractive Index of 1.5203.

In order for a material to function as a one-piece IOL, it must have a certain degree of resiliency which allows the haptics to maintain the IOL in the ocular environment. In order to achieve this resiliency, the modulus of the material must be sufficiently high such that even at the elevated temperature in the ocular environment (35°C), the material retains sufficient rigidity and resiliency. However this rigidity increases the likelihood that the IOL may become damaged when passed through an inserter cartridge. The present inventors have surprisingly found that an IOL fabricated according to the teachings of the present invention and having a high modulus and a low percent elongation can pass through an inserter cartridge without damaging either itself or the cartridge tube.

Thus, disclosed herein are biocompatible polymeric compositions surprisingly useful in fabricating intraocular lenses intended for implantation into the posterior chamber of both phakic and aphakic eyes. Moreover the PC-IOLs made in accordance with the teachings of the present invention have a refractive index (n_{D}) at 20°C-25°C of between approximately 1.45 and 1.55. In a preferred embodiment the PC-IOL has a refractive index (n_{D}) at 20°C-25°C of approximately 1.52.

The biocompatible polymeric compositions of the present invention provide uniquely balanced properties that make them especially useful in fabricating thin, pliable PC-IOLs that have excellent mechanical strength and durability. The PC-IOLs made having the physical characteristics disclosed above will be pliable enough to be easily folded, rolled or other wise deformed sufficiently for insertion through small incisions, have the mechanical strength necessary to absorb incidental impact after implantation and be strong enough to permit the lenses to be sufficiently thin to fit comfortably within the phakic eye's posterior chamber and while being suitable for correcting myopia, hyperopia, presbyopia, astigmatisms and for implantation after removal of the natural crystalline lens as warranted by medical conditions such as cataracts.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a" and "an" and "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

## Claims

1. A biocompatible polymer comprising:
52 mass percent to 56 mass percent of a first alkyl acrylate,
18 mass percent to 22 mass percent of a second alkyl acrylate,
24 mass percent to 28 mass percent of a third alkyl acrylate, and
3 mass percent to 5 mass percent of a diacrylate ester cross-linking agent;
wherein said biocompatible polymer is used to form a posterior chamber intraocular lens and
wherein said first alkyl acrylate is phenoxyethyl acrylate, said second alkyl acrylate is ethyl acryate and said third alkyl acrylate is ethyl methacrylate.

2. The biocompatible polymer according to claim 1 wherein said diacrylate ester cross-linking agent is selected from the group consisting of ethylene glycol dimethacrylate, propylene glycol dimethacrylate, ethylene glycol diacrylate and combinations thereof.

3. The biocompatible polymer according to claim 2 wherein said diacrylate ester cross-linking agent is ethyleneglycol dimethacrylate.

4. The biocompatible polymer according to claim 1 wherein:
said first alkyl acrylate is present in a mass percent of 54 mass percent;
said second alkyl acrylate is present in a mass percent of 20 mass percent;
said third alkyl acrylate is present in a mass percent of 26 mass percent; and
said diacrylate ester crosslinking agent is present in a mass percent of 4 mass percent;
wherein residual solvents and W absorbing compounds make up the remaining mass percentage such the total mass percent is 100.

5. The biocompatible polymer according to claim 1 or 3, further comprising at least one ultraviolet (UV) light absorbing compound.

6. The biocompatible polymer according to claim 5, further comprising a blue-violet light absorbing compound.

7. The biocompatible polymer according to claim 1 wherein said biocompatible polymer has a tensile strength of 10.75 MPa (1559 psi), an elongation at break of 97% and is used to form a posterior chamber intraocular lens.

8. The biocompatible polymer according to claim 7 wherein said posterior chamber intraocular lens has a refractive index of between 1.45 and

9. The biocompatible polymer according to claim 8 wherein said posterior chamber intraocular lens has a refractive index of 1.52.

## Patentansprüche

1. Biokompatibles Polymer, umfassend:
52 Massen% bis 56 Massen% eines ersten Alkylacrylats,
18 Massen% bis 22 Massen% eines zweiten Alkylacrylats,
24 Massen% bis 28 Massen% eins dritten Alkylacrylats und
3 Massen% bis 5 Massen% eines Diacrylatester-Vernetzungsmittels,
wobei das biokompatible Polymer zur Herstellung einer intraokularen Linse der hinteren Augenkammer verwendet wird und worin das erste Alkylacrylat Phenoxyethylacrylat ist, das zweite Alkylacrylat Ethylacrylat und das dritte Alkylacrylat Ethylmethacrylat ist.

2. Biokompatibles Polymer gemäß Anspruch 1, worin das Diacrylatester-Vernetzungsmittel ausgewählt ist aus der Gruppe bestehend aus Ethylenglycoldimethacrylat, Propylendimethacrylat, Ethylenglycoldiacrylat und Kombinationen davon.

3. Biokompatibles Polymer gemäß Anspruch 2, worin das Diacrylatester-Vernetzungsmittel Ethylenglycoldimethacrylat ist.

4. Biokompatibles Polymer gemäß Anspruch 1, worin das erste Alkylacrylat in einem Massenantil von 54 Massen% vorliegt,
das zweite Alkylacrylat in einem Massenantil von 20 Massen% vorliegt,
das dritte Alkylacrylat in einem Massenantil von 26 Massen% vorliegt, und
das Diacrylatester-Vernetzungsmittel in einem Massenanteil von 4 Massen% vorliegt,
wobei verbleibende Lösungsmittel und UV-absorbierende Verbindungen den verbleibenden Massenanteil ausmachen, so dass die gesamten Massenprozente 100 betragen.

5. Biokompatibles Polymer gemäß Anspruch 1 oder 3 ferner umfassend wenigstens eine Ultraviolett-(UV)-lichtabsorbierende Verbindung.

6. Biokompatibles Polymer gemäß Anspruch 5 ferner umfassend eine ein blauviolettes Licht absorbierende Komponente.

7. Biokompatibles Polymer gemäß Anspruch 1, wobei das biokompatible Polymer eine Zugfestigkeit von 10,75 MPa (1.559 psi), eine Bruchdehnung von 97 % aufweist und zur Herstellung einer intraokularen Linse der hinteren Augenkammer verwendet wird.

8. Biokompatibles Polymer gemäß Anspruch 7, worin die Intraokularlinse der hinteren Augenkammer einen Brechungsindex zwischen 1,45 und 1,55 aufweist.

9. Biokompatibles Polymer gemäß Anspruch 8, worin die Intraokularlinse für die hintere Augenkammer einen Brechungsindex von 1,52 aufweist.

## Revendications

1. Polymère biocompatible comprenant :
52 pour cent en masse à 56 pour cent en masse d'un premier acrylate d'alkyle,
18 pour cent en masse à 22 pour cent en masse d'un deuxième acrylate d'alkyle,
24 pour cent en masse à 28 pour cent en masse d'un troisième acrylate d'alkyle, et
3 pour cent en masse à 5 pour cent en masse d'un agent de réticulation ester diacrylate ;
dans lequel ledit polymère biocompatible est utilisé pour former une lentille intraoculaire de la chambre postérieure et
dans lequel ledit premier acrylate d'alkyle est l'acrylate de phénoxyéthyle, ledit deuxième acrylate d'alkyle est l'acrylate d'éthyle et ledit troisième acrylate d'alkyle est le méthacrylate d'éthyle.

2. Polymère biocompatible selon la revendication 1, dans lequel ledit agent de réticulation ester diacrylate est sélectionné parmi le groupe composé de diméthacrylate d'éthylène glycol, de diméthacrylate de propylène glycol, de diacrylate d'éthylène glycol et de leurs combinaisons.

3. Polymère biocompatible selon la revendication 2 dans lequel ledit agent de réticulation ester diacrylate est le diméthacrylate d'éthylène glycol.

4. Polymère biocompatible selon la revendication 1, dans lequel :
ledit premier acrylate d'alkyle est présent en un pourcentage en masse de 54 pour cent en masse ;
ledit deuxième acrylate d'alkyle est présent en un pourcentage en masse de 20 pour cent en masse ;
ledit troisième acrylate d'alkyle est présent en un pourcentage en masse de 26 pour cent en masse ; et
ledit agent de réticulation ester diacrylate est présent en un pourcentage en masse de 4 pour cent en masse ;
dans lequel des solvants résiduels et des composés absorbant l'UV forment le pourcentage en masse restant de sorte que le pourcentage en masse total soit de 100.

5. Polymère biocompatible selon la revendication 1 ou 3 comprenant en outre au moins un composé absorbant la lumière ultraviolette (UV).

6. Polymère biocompatible selon la revendication 5 comprenant en outre un composé absorbant la lumière bleu-violette.

7. Polymère biocompatible selon la revendication 1 dans lequel ledit polymère biocompatible a une résistance à la traction de 10,75 MPa (1559 psi), un allongement à la rupture de 97% et est utilisé pour former une lentille intraoculaire de chambre postérieure.

8. Polymère biocompatible selon la revendication 7 dans lequel ladite lentille intraoculaire de chambre postérieure présente un indice de réfraction compris entre 1,45 et 1,55

9. Polymère biocompatible selon la revendication 8 dans lequel ladite lentille intraoculaire de chambre postérieure présente un indice de réfraction de 1,52.
